(19) Europäisches Patentamt
European Patent Office
Office européen des brevets

(11) Publication number: 0 302 541 A2

(12) # EUROPEAN PATENT APPLICATION

(21) Application number: 88201433.5

(22) Date of filing: 07.07.88

(51) Int. Cl.4: C07D 491/052 , C07D 307/16 , C07D 309/30 , C07D 309/32 , //(C07D491/052,311:00,209:00)

(30) Priority: 06.08.87 IT 2159387

(43) Date of publication of application: 08.02.89 Bulletin 89/06

(84) Designated Contracting States: AT BE CH DE ES FR GB GR IT LI LU NL SE

(71) Applicant: BLASCHIM S.p.A.
Via Vittor Pisani, 28
I-20124 Milano(IT)

(72) Inventor: Citterio, Attilio
Piazzale Piola, 5
I-20131 Milano(IT)
Inventor: Fancelli, Daniele
Via Giannella, 21
I-20152 Milano(IT)
Inventor: Pesce, Luca
Via Rossini, 20
I-20050 Monza Milano(IT)

(74) Representative: Marchi, Massimo et al
c/o Marchi & Mittler s.r.l. Viale Lombardia 20
I-20131 Milano(IT)

(54) **A method of preparing ethodolac.**

(57) A method of preparing ethodolac by reacting furan with a reactive derivative of propionic acid, reacting 2-propionyl furan with an ester of a haloacetic acid oxidising an ester of 3-hydroxy-3(2′-furyl)-pentanoic acid protecting the hydroxy group of the resulting pyran derivative and hydrogenolysis and reduction therof, condensation of 5,6-dihydro-2-ethyl-2-carboxymethyl-2H-pyran(4H)-one or an ester thereof with 2-ethylphenylhydrazine and finally, when the resulting product is an ester, hydrolysis thereof.

EP 0 302 541 A2

## A Method of Preparing Ethodolac

The invention relates to a method of preparing ethodolac and some intermediates of use in the method.

Ethodolac is a known anti-inflammatory drug, the chemical name of which is 1,8-diethyl-1,3,4,9-tetrahydropyran[3,4b]-indolyl-1-acetic acid.

No method for specifically producing ethodolac has ever been described in the literature. US P 3 843 681 describes a general method which could also be used to prepare ethodolac, but the examples refer to analogous products and ethodolac is not among the products given as examples. Ethodolac is mentioned for the first time by C.A. Demerson et al (J. Med. Chem. 19, 391-395, 1976) but in this case also there is no description of the specific method of synthesis.

The general method described in both the aforementioned documents mainly comprises condensing a tryptofol having the formula

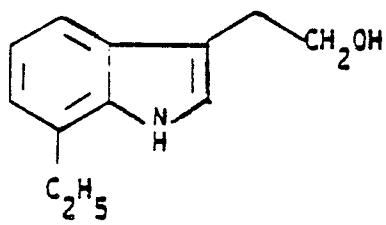

(A)

with an ester of propionylacetic acid having the formula $C_2H_5$-CO-$CH_2$-COOR (B)
where R is a low molecular-weight alkyl, in the presence of such an acid catalyst such as p-toluene sulphonic acid, boron trifluoride or phosphorus pentoxide.

In a summarising table in the aforementioned article by C.A. Demerson et al, the authors state that they prepared ethodolac with a yield of more than 90% by the method specified by them and described in connection with the preparation of the analogous 1-ethyl-8-(2-propyl)-1,3,4,9-tetrahydropyran [3,4b]- indolyl -1-acetic acid. In reality, when operating in accordance with the teaching of the aforementioned specified method, it is impossible to obtain more than 50% ethodolac.

In addition to this undoubtedly important aspect, the main disadvantage of this method is that tryptofol of formula (A) is very expensive and difficult to purify.

Another serious disadvantage of the method indicated hereinbefore is the increasing importance of the S-(+) enantiomer of ethodolac, which bas been found to be solely responsible for the pharmacological activity whereas the R-(-) enantiomer is completely inactive (US PSS 4 501 899, 4 515 961, 4 520 203 and 4 544 757).

The asymmetric carbon atom is marked by an asterisk in the following formula:

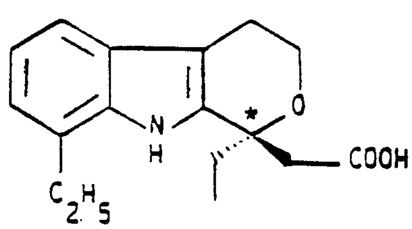

(V)

As can be seen, in the known method of synthesis the centre of asymmetry is formed only in the last step and without it being possible to induce the formation of the single desired enantiomer.

It is therefore necessary first to prepare racemic ethodolac, to separate the desired S-(+) enantiomer and destroy the inactive R-(-) enantiomer. All this greatly increases the cost of the S-(+) enentiomer.

The invention aims to obviate the aforementioned disadvantages by a method which avoids the use of tryptofol of formula (A) and in which the asymmetric carbon atom is formed in one of the first steps of the process via a type of reaction which can also be carried out stereospecifically. Alternatively the intermediate containing the asymmetric carbon atom can be prepared in racemic form and then separated by forming suitable diastereoisomers. In this case also there is a considerable economic advantage since the

intermediate is prepared during one of the first steps and its cost is very low, so that the separation and destruction of the optically active form leading to the R-(-) enantiomer of ethodolac will have only a slight effect on the cost of preparing S-(+) ethodolac.

It has now been found that the aforementioned objectives can be obtained by the following synthesis:

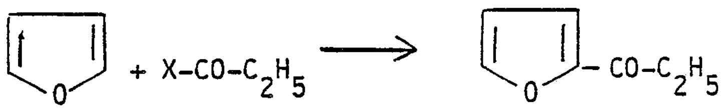

(I)

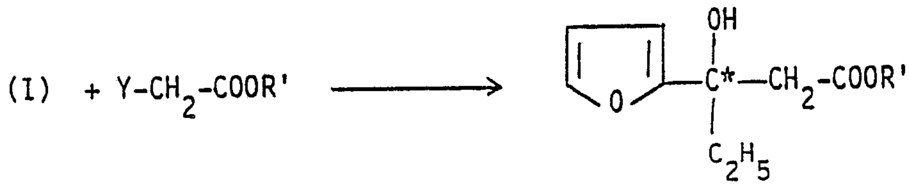

(II)

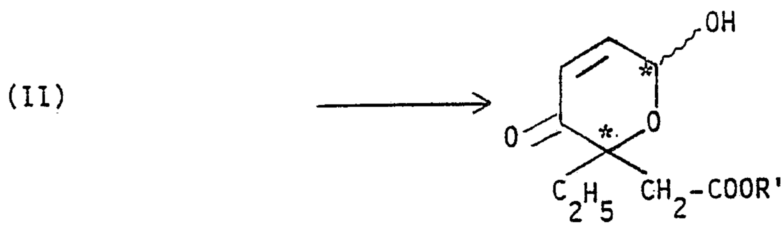

(III)

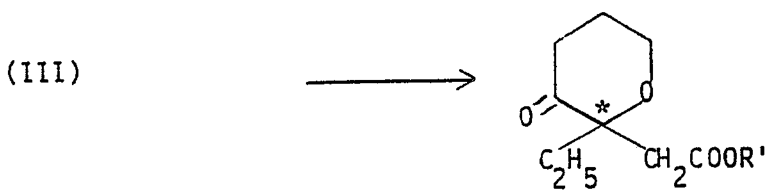

(IV)

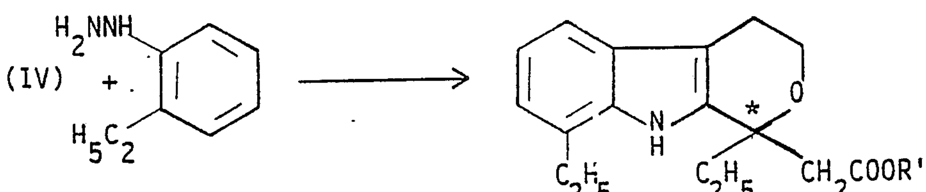

(V-bis)

where X is chlorine, bromine or-O-CO-$C_2H_5$

Y is chlorine or bromine and

R′ is hydrogen or alkyl with 1-6 carbon atoms or benzyl or the radical of an optically active alcohol.

The invention therefore relates to a method of preparing ethodolac comprising:

(a) reacting furan with a reactive derivative of propionic acid;

(b) reacting the resulting 1-propionyl furan with an ester of a haloacetic acid;

(c) oxidising the ester of 3-hydroxy-3-(2′-furyl)pentanoic acid;

(d) protecting the hydroxy group of the resulting pyran derivative, followed by hydrolysis and reduction, and

(e) condensing 5,6-dihydro-2-ethyl-2-carboxymethyl-2H-pyran(4H)-one or an ester thereof with 2-ethylphenyl-hydrazine or an acid addition salt thereof and, when the resulting product is an ester, the ester is hydrolysed.

Phase (a) is preferably brought about by reacting furan with the bromide or chloride or anhydride of propionic acid in the presence of a suitable catalyst or a suitable diluent at a temperature between -15 °C and the reflux temperature of the reaction mixture.

The following are examples of suitable catalysts: boron trifluoride, zinc chloride and phosphoric acid when the acylating agent is the anhydride, or aluminium chloride when the acylating agent is the acid chloride or bromide.

Examples of suitable diluents are: carbon disulphide, methylene chloride or an excess of the anhydride when used as the acylating agent.

Phase (b) is preferably brought about by reacting 2-propionyl furan with an ester of bromo- or chloroacetic acid and an aliphatic alcohol containing 1-8 carbon atoms, benzyl alcohol or an optically active alcohol in the presence of zinc and a suitable diluent and at a temperature between 0 °C and 115 °C.

Examples of suitable optically active alcohols are (-) borneol and (-) menthol.

Zinc can be used in powder or granule form. Examples of suitable diluents are aromatic hydrocarbons such as benzene and toluene.

It is preferable to operate at the reflux temperature of the diluent used.

The reaction can be continuous or discontinuous.

If desired, the resulting ester can be hydrolysed to give 3-hydroxy-3-(2′furyl)-pentanoic acid.

Phase (c) is preferably brought about when cold, using a suitable oxidising agent. Examples of suitable oxidising agents are peracids such as m-chloroperbenzoic acid or permaleic acid or halogens such as bromine and chlorine. It is preferable to operate at a temperature between -35 °C and 20 °C and in the presence of a suitable diluent such as methanol or methylene chloride.

Step (d) is preferably brought about by first protecting the hydroxyl group. Examples of suitable protecting agents are organic halogen carbonates having the formula:

Y-O-CO-O-R″

where Y is chlorine or bromine and
R″ is an alkyl with 1-6 carbon atoms or benzyl.
R″ preferably stands for methyl, propyl, butyl or benzyl.

The reaction with the protecting agents is usually brought about when cold, preferably at a temperature between 0 °C and ambient temperature, in the presence of an organic or inorganic base able to capture the hydrohalic acid which forms during the reaction.

Examples of suitable bases are alkali-metal and alkaline earth carbonates and bicarbonates, tertiary aliphatic amines such as triethylamine and trimethylamine, or secondary cyclic amines such as piperidine.

The second part of step (d) (hydrogenolysis and reduction) is preferably brought about with hydrogen in the presence of a suitable catalyst such as palladium on carbon in an autoclave at a temperature between 20 °C and 60 °C and a pressure between 1 and 30 atmospheres.

When the resulting product is an ester it can if required be hydrolysed to give 5,6-dihydro-2--ethyl-2-carboxymethyl-2H-pyran-(4H)-one.

Finally, step (e) is preferably brought about with heating in a nitrogen atmosphere in the presence of a suitable diluent such as trifluoroethanol or ethanol or other low molecular-weight alcohols.

The intermediates obtained during the aforementioned process are novel. They therefore constitute another subject to the invention.

More particularly, compounds are novel which have the following formula:

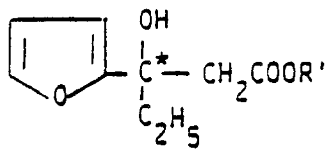

(II)

and

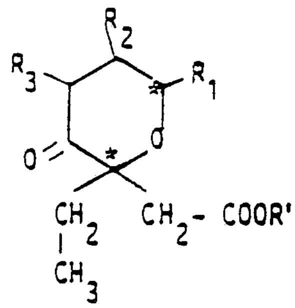

(VI)

where $R_1$ is hydrogen, hydroxyl, or -O-CO-OR″, wherein
R″ is alkyl with 1-6 carbon atoms or benzyl;
$R_2$ and $R_3$ are hydrogen or together form a covalent bond, and
R′ is hydrogen or an alkyl with 1-8 carbon atoms or benzyl or the radical of an optically active alcohol.

As indicated hereinbefore by an asterisk, compounds (II) have an asymmetric carbon atom whereas compounds of formula (VI) have one asymmetric carbon atom when $R_1$ is hydrogen and two when $R_1$ is OH or -O-CO-OR″.

The aim of the invention therefore is to protect the individual enantiomers and epimers and their racemic mixtures.

The enantiomers of formula (II) can be prepared by the methods indicated hereinbefore, by reacting the compound of (I) with (i) an ester of haloacetic acid and an optically active alcohol such as (-) borneol or (-) menthol, or (ii) with an ester of a haloacetic acid and a non-optically active alcohol in the presence of a suitable optically active catalyst.

Examples of suitably optically active catalyst are substituted ethanolamines such as 1-alkyl- or 1-aryl-1-hydroxy-ethylamine, 1-alkyl- or 1 aryl-2-amino-ethanols and 1-alkyl- or 1-aryl-2-amino-1,3-propanediols. Typical examples are catecholamine, cinchonidine, quinine and (+) 2-amino-butanol.

When the resulting product is not optically pure, the desired enantiomer can be isolated by conventional methods such as fractional crystallisation or liquid-liquid chromatography.

The desired optically pure enantiomer can then be used in the subsequent steps (c), (d) and (e) indicated hereinbefore, since these steps do not include racemising conditions.

Optically active compounds of formula (VI) can be obtained from compounds of formula (II) as indicated hereinbefore or by separating the racemic mixtures with optically active alcohols or bases.

Separation with optically active alcohols can be brought about by conventional methods by esterification of a compound of formula (VI) in which R′ is hydrogen or by transesterification of a compound of formula (VI) in which R′ is an alkyl with 1-8 carbon atoms or benzyl.

The resulting substances are mixtures of diastereoisomers which can be separated by conventional methods such as fractional crystallisation or liquid-liquid chromatography to give the desired enantiomer or epimer.

Separation with optically active bases can be brought about, also by known methods, by salification of a compound of (VI) in which R′ is hydrogen, followed by separation of the resulting diasteroisomeric salts.

When $R_1$ is hydroxyl or -O-CO-OR″, the desired optically active compounds or formula (VI) can be used in the subsequent steps (d) and (e) without racemisation.

The same applies to compounds of formula (VI) in which $R_1$, $R_2$ and $R_3$ are hydrogen when used in the subsequent step (e).

The following examples illustrate the invention without limiting it in any way.

## EXAMPLE 1

2-Propionyl furan

Boron trifluoride etherate (25.2 ml; 0.2 mols) was added dropwise to a mixture of furan (136 g; 2 mols) and propionic anhydride (299 g; 2.3 mols) at -10° C.

The internal temperature which has risen to 15° C was brought to 0° C in 10-15 minutes. Cooling was stopped and the temperature of the reaction mixture was allowed to rise to ambient temperature. The reaction mixture was kept at this temperature with agitation for 30 minutes and then poured slowly into a mixture of 30% sodium hydroxide (400 ml) and ethyl ether (100 ml) kept under agitation at -10° 0° C.

The organic phase was separated, washed with water, dehydrated and distilled firstly at atmospheric pressure until the solvent had been removed and then in vacuo.

The fraction boiling at 72° C-75° C (8 mm Hg) was collected, yielding a white solid,

m.p. 28-29° C. Yield: 198 g (80%).

EXAMPLE 2

Ethyl 3-hydroxy-3-(2′-furyl)-pentanoate

The apparatus described in J.0.C. 39, 269. 1974 was loaded with granular zinc (20 mesh; 110 g) and benzene (15 ml). After reflux-heating the benzene, a solution of 2-propionyl furan (50g; 0.40 mols) and ethyl bromoacetate (90 ml; 0. 8 mols) was added to the fixed bed at a speed of 1.8 ml/min. Heating was continued with slight reflux. A red solution flowed from the column into a 1-litre flask and was collected and poured into a mixture of 15% sulphuric acid (850 ml) and ethyl ether (100 ml) kept under agitation at 0° 5° C. Agitation was continued for 15 minutes and then the reaction mixture was left to warm up to ambient temperature. The organic phase was separated from the aqueous phase. which was extracted with ethyl ether (3 x 50 ml). The combined organic phases were washed with water (100 ml), with a 2% aqueous solution of sodium bicarbonate (100 ml) and again with water to neutrality. The organic phase was then dehydrated with sodium sulphate and evaporated in vacuo. The residue was distilled in vacuo (0.8 mm Hg), collecting the fraction which boils at 80° C-83° C.

Yield, 79 g (92%) $IR\nu_{max}$ : 3,500 (OH); 1715 (CO ester ); 1172 (C-O)
NMR ($CDCl_3$,delta) : 0.83 (t,3H,$CH_2$-$CH_3$); 1.20 (t,3H,O-$CH_2$-$C\underline{H}_3$); 1.84 (q,2H, $C\underline{H}_2$-$CH_3$); 2.68 (d,1H,$C\underline{H}_2$COOR); 2.98 (d,1H,$C\underline{H}_2$-COOR); 4.10 (q,2H,O-$C\underline{H}_2$-$CH_3$); 4.33 (s,1H,OH); 6.3 (m,2H,$H_3$′,4′); 7.3 (m,1H,$H_5$′)

The following were prepared in a similar manner:
The methyl ester,
The benzyl ester,
The isoamyl ester and
The n-heptyl ester.

EXAMPLE 3

2-ethoxycarbonylmethyl-2-ethyl-6-hydroxy-2H-pyran-3(6H) -one

The compound obtained in Example 2 (42 g; 0.198 mols) was dissolved in methylene chloride (300 ml). The solution was cooled to 0° C/5° C and m-chloro-perbenzoic acid (34.1 g; 0.248 mols) was added in small portions. The mixture, after being kept under agitation at 5° C-10° C for 16 hours, was filtered. The solution was successively washed with a 5% solution of sodium iodide (4x50 ml), a 5% solution of sodium thiosuphate (2x100 ml), a 5% solution of sodium bicarbonate (3x100 ml) and finally with water (2x100 ml);

The organic phase was separated, dehydrated over sodium sulphate, evaporated in vacuo and dried. The residue contained 83% of the desired product (85% yield of the compound in Example 1).

The crude product was purified by chromatography on a silica gel column (hexane:ethyl acetate 9:1).

The desired product was thus obtained in the form of an oil (mixture of the two $C_6$ epimers).

IR,$\nu_{max}$ : 3450 (OH), 1680 (CO, conjugate), 1715 (CO, ester ).
NHR ($CDCl_3$,delta) : 0.9 (2t,3H,$CH_2$-C$\underline{H}_3$); 1.25 (2t,3H, O-$CH_2$ -C$\underline{H}_3$); 1.8 (m,2H,C$\underline{H}_2$-$CH_3$); 2.7 (d,d,1H,C$\underline{H}_2$ -COOH);3.1 (d,d,1H,C$\underline{H}_2$COOEt); 4 (wide,1H, O$\underline{H}$); 4.13 (2q, O-C$\underline{H}_2$-$CH_3$ ); 5.72 (m,1H,$H_6$); 6.19 (d,1H,$H_4$); 6.92 (m,1H,$H_5$).

EXAMPLE 4

2-ethoxycarboxylmethyl-2-ethyl-6-hydroxy-2H-pyran-3(6H)-one ethyl carbonate

Ethyl chlorocarbonate (65 g; 0.6 mols) was added slowly dropwise and with agitation to a mixture of the compound in Example 3 (86 g; 0.48 mols) in anhydrous ethyl ether (800 ml) at O° C, after previously adding triethylamine (61 g; 0.6 mols). At the end of the addition process, the reaction mixture was kept under agitation at 0° C/5° C for 90 minutes and at ambient temperature for a further 60 minutes. After removing the solid phase by filtration, the ethereal phase was washed with water (2x100 ml), with 2% hydrochloric acid (2x100 ml) and finally with water (100 ml). The solution was dehydrated on sodium sulphate and the solvent was evaporated at reduced pressure at 20° C. The residue was chromatographed on a silica gel column (hexane:ethyl acetate 80:20) thus obtaining the desired product in oil form (mixture of two $C_6$ epimers).

IR $\nu_{max}$ : 1715 (CO ester), 1683 (CO conjugate), 1745 (CO carbonate).
NMR ($CDCl_3$, delta) : 0.94 and 0.96(t,3H,$CH_2$-C$\underline{H}_3$); 1.22 and 1.39 (2t,6H,O-$CH_2$-C$\underline{H}_3$); 1.9 (m,2H, C$\underline{H}_2$-$CH_3$ ; 2.75 and 2.78 (d,1H,C$\underline{H}_2$ -COO-Et); 3.07-3.11 (d,1H,C$\underline{H}_2$-$COOC_2H_5$); 4.10 (q,2H,O-C$\underline{H}_2$-$CH_3$); 4.30 (q,2H, O-COC$\underline{H}_2$-$CH_3$), 6.3 (d,1H,$H_4$); 6,51 (m,1H,$H_6$); 6.8 and 6.9 (d,d,1H,$H_5$).

EXAMPLE 5

5,6-dihydro-2-ethyl-2-carbethoxylmethyl-2H-pyran(4H)-one

The compound in Example 4, freshly prepared (5.5 g; 19 mols), was dissolved in ethyl acetate (200 ml). After adding 10% palladium on carbon (2 g) the mixture was hydrogenated in an autoclave at 5 atmospheres and at 50° C for 4 hours. The mixture was then cooled and filtered. The solution was distilled in vacuo and the fraction boiling at 94° C-96° C (0.6 mm Hg) was collected.

yield : 3. 4 g (90%)

IR$\nu_{max}$ : 1715 (CO ester ), 1735 (CO ketone)
H-NMR($CDCl_3$, delta) : 0.9 (t,3H,$CH_2$-C$\underline{H}_3$); 1.24 (t,3H,O-$CH_2$C$\underline{H}_3$); 1.6 (m,2H,C$\underline{H}_2$-$CH_3$); 2-2.7 (m, 4H, CO-C$\underline{H}_2$ -C$\underline{H}_2$); 2.52 (d,1H,C$\underline{H}_2$ COOR); 2.98 (d,1H,-$CH_2$-COOR); 4.84 (m,2H,O-C$\underline{H}_2$-$CH_2$-); 4.1 (q,2H,O-C$\underline{H}_2$$CH_3$).

The following were prepared in similar manner:
The methyl ester, b.p. 116° C-118° C (3mm Hg):
The isoamyl ester, b.p. 121° C-122° C (0.5mm Hg);
The n-heptyl ester, b.p. 132° C-133° C (0.1mm Hg).

The methyl ester was hydrolysed with sodium hydroxide in methyl alcohol with reflux for an hour. giving 5,6-dihydro-2-ethyl-2-carboxymethyl-2H-pyran-3-(4H)-one, m.p. 131° C-134° C (chloroform/pentane). The same substance can be obtained by alkaline hydrolysis and subsequent acidification of the hydrogenated solution in an autoclave, of the corresponding benzyl ester, cooled and filtered.

EXAMPLE 6

1.8-diethyl-1-1,3,4,9-tetrahydropyran[3,4b]-indolyl-1-acetic acid

The methyl ester (0.6 g; 0.31 mols) in Example 5 was added under nitrogen to the hydrochloride of 2-ethylphenylhydrazine(5.18 g; 30 mols) in trifluoroethanol (60 ml).

The reaction mixture was reflux-heated under nitrogen and some more methyl ester (6 g; 3.1 mols) was added dropwise. The mixture was kept at gentle reflux under nitrogen for a further four hours and then the solvent was removed by distillation in vacuo.

The residue was chromatographed on a silica gel column (hexane:ethyl acetate 8:2), thus yielding the methyl ester of ethodolac.

m.p. 130° C-131° C; Yield 4.8g (53%)

This ester was hydrolysed with 30% sodium hydroxide (12ml) in methanol (80 ml) with reflux for 2 hours. The solvent was eliminated by evaporation and the residue was dissolved in water (50 ml) and in methylene chloride (20 ml). The organic phase was discharged and the aqueous layer was brought to pH 4 with concentrated hydrochloric acid and extracted with toluene. The organic layer was separated and the solvent was eliminated by evaporation. The residue was dissolved in hot chloroform (3 ml) and hexane (10 ml) was then added. Finally the mixture was reflux-heated.

Ethodolac was obtained by cooling.

m.p. 145° C-147° C (chloroform/hexane).

**Claims**

1. A method of preparing ethodolac characterised in that (a) furan is reacted with a reactive derivative of propionic acid, (b) the resulting 2-propionyl furan is reacted with an ester of a halo-acetic acid, (c) the ester of 3-hydroxy-3-(2′-furyl)-pentaoic acid is oxidised, (d) the hydroxy group of the resulting pyran derivative is protected for subsequent hydrogenolysis and reduction, and (e) 5,6-dihydro-2-ethyl-2carboxymethyl-2H -pyran (4H)-one or an ester thereof is condensed with 2-ethyl-phenylhydrazine or an acid addition salt thereof and, when the resulting product is an ester, it is hydrolysed.

2. A method according to claim 1,characterised in that in step (a), the furan is reacted with the bromide, chloride or anhydride of propionic acid in the presence of a suitable catalyst and a suitable solvent at a temperature between -15° C and the boiling point of the reaction mixture.

3. A method according to claim 2, characterised in that the catalyst is boron trifluoride, zinc chloride and phosphoric acid when the acylating agent is the anhydride, or aluminium trichloride when the acylating agent is the bromide or chloride of propionic acid.

4. A method according to claim 2 or 3, characterised in that the solvent is carbon sulphide or methylene chloride or an excess of the anhydride when used as the acylating agent.

5. A method according to any of the preceding claims 1 to 4, characterised in that in step (b) 2-propionyl furan is reacted with an ester of bromo- or chloroacetic acid and an aliphatic alcohol or an optically active alcohol, in the presence of zinc and a suitable diluent at a temperature between 0° C and 115° C.

6. A method according to claim 5, characterised in that the diluent is an aromatic hydrocarbon.

7. A method according to claim 5 or 6, characterised in that the reaction is brought about in the presence of a suitable optically active catalyst.

8. A method according to any of the preceding claims from 1 to 7, characterised in that in step (c) the operating temperature is between -35° C and 20° C.

9. A method according to any of the preceding claims from 1 to 8, characterised in that the oxidising agent used in step (c) is a peracid or halogen.

10. A method according to claim 9, characterised in that the oxidising agent is m-chlorobenzoic acid or permaleic acid or bromine or chlorine.

11. A method according to any of the preceding claims from 1 to 10, characterised in that the first part of step (d) the hydroxy group is protected by an organic halocarbonate.

12. A method according to claim 11, characterised in that the hydroxyl group is protected by the organic halocarbonate at a temperature between 0° C and ambient temperature.

13. A method according to claim 11 or 12, characterised in that the organic halocarbonate is methyl or propyl or butyl or benzyl chlorocarbonate.

14. A method according to any of the preceding claims from 1 to 13, characterised in that the second part of step (d) is brought about with hydrogen in the presence of a catalyst at a pressure between 1 and 30 atmospheres and at a temperature between 20° C and 60° C.

15. A method according to any of the preceding claims from 1 to 14, characterised in that step (e) is brought about with heating in a nitrogen atmosphere in the presence of a suitable diluent.

16. A method according to claim 15, characterised by operating at the reflux temperature of the reaction mixture.

17. A method according to claims 15 and 16, characterised in that the diluent is a low molecular-weight alcohol.

18. Compounds having the formula

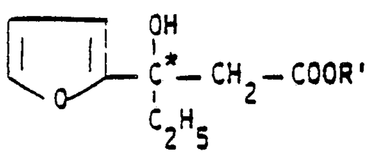

(II)

in which R′ is hydrogen or alkyl with 1-8 carbon atoms or benzyl or the radical of an optically active alcohol.

19. Compounds having the formula

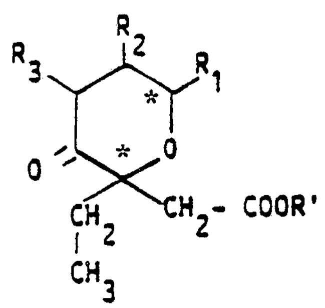

(VI)

where $R_1$ is hydrogen, hydroxy or -O-CO-OR″, wherein
R″ is alkyl with 1-6 carbon atoms or benzyl;
$R_2$ and $R_3$ are hydrogen or together form a covalent bond, and
R′ is hydrogen, alkyl with 1-8 carbon atoms, benzyl or the radical of an optically active alcohol.

20. A method of producing S(+)- ethodolac characterised in that the S(+)- enantiomer of a 3-hydroxy-3-(2′-furyl)-pentanoic acid ester is oxidised to provide a pyran derivative, the hydroxy group of which is then protected; the pyran derivative is then subjected to hydrogenolysis and reduction to provide 5,6-dihydro-2-ethyl-2-carboxymethyl-2H-pyran(4H)-one this product or an ester thereof is then condensed with 2-ethyl-phenylhydrazine or an acid addtition salt thereof and, where the resulting product is an ester, it is hydrolysed.